(19) Europäisches Patentamt
European Patent Office
Office européen des brevets

(11) Veröffentlichungsnummer: **0 288 847 B1**

(12) **EUROPÄISCHE PATENTSCHRIFT**

(45) Veröffentlichungstag der Patentschrift: **09.12.92**

(51) Int. Cl.$^5$: **C07H 3/02**

(21) Anmeldenummer: **88106103.0**

(22) Anmeldetag: **16.04.88**

# (54) Kontinuierliches Verfahren zur Epimerisierung von Zuckern, insbesondere von D-Arabinose zu D-Ribose.

(30) Priorität: **30.04.87 DE 3714473**

(43) Veröffentlichungstag der Anmeldung:
**02.11.88 Patentblatt 88/44**

(45) Bekanntmachung des Hinweises auf die Patenterteilung:
**09.12.92 Patentblatt 92/50**

(84) Benannte Vertragsstaaten:
**CH DE FR GB IT LI NL**

(56) Entgegenhaltungen:
**EP-A- 0 020 959**
**FR-A- 2 311 846**

**CHEMICAL ABSTRACTS, Band 97, Nr. 13, 27. September 1982, Seite 647, Zusammenfassung Nr. 110335y, Columbus, Ohio, US; & JP-A-82 54 198 (TOWA KASEI KOGYO CO., LTD) 31-03-1982 (Kat. D)**

(73) Patentinhaber: **BASF Aktiengesellschaft**
**Carl-Bosch-Strasse 38**
**W-6700 Ludwigshafen(DE)**

(72) Erfinder: **Dobler, Walter, Dr.**
**Wilhelm-Blum-Strasse 4**
**W-6900 Heidelberg(DE)**
Erfinder: **Paust, Joachim, Dr.**
**Ringstrasse 3**
**W-6708 Neuhofen(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein kontinuierliches Verfahren zur Epimerisierung von D- und L-Zuckern mit transständigen OH-Gruppen in 2- und 3-Stellung des Zuckers, insbesondere von D-Arabinose zu D-Ribose. Die D-Ribose wird für die Synthese von Vitamin B 2 benötigt.

Es ist bekannt, daß D-Ribose durch Extraktion aus Naturstoffen, durch Fermentation eines Mikroorganismus oder durch chemische Synthese aus Furan oder Glucose hergestellt werden kann. Alle diese Verfahren zeichnen sich jedoch dadurch aus, daß sie kompliziert sind und eine geringe Ausbeute ergeben.

Die Herstellung von D-Ribose wurde lange Zeit technisch nach einem Verfahren durchgeführt, bei dem D-Glucose zur Bildung von D-Arabinose mit Sauerstoff in einer wäßrigen Alkali-Lösung oxidiert und diese in Form eines Metallsalzes wie z. B. von Quecksilber oder Zinksalz abgetrennt, zu D-Ribonolacton lactonisiert und mit Natriumamalgam zu D-Ribose reduziert wurde. Die Erwärmung von D-Arabinose in einer wäßrigen Alkalilösung ergibt eine Mischung, die ein Gleichgewichtsverhältnis von D-Arabonsäure zu D-Ribonsäure von 70 : 30 aufweist. Es ist hierbei unmöglich, einen Anteil von D-Ribonsäure zu erhalten, der 30 % übersteigt. Schwierigkeiten ergaben sich für das Verfahren außerdem durch die Verwendung der großen Mengen an Quecksilber für die Amalgam-Reduktion.

Bilik et al. berichteten über die Epimerisierbarkeit von verschiedenen Sacchariden in wäßriger Lösung in Gegenwart eines Molybdänsäure-Katalysators, einschließlich der Epimerisierbarkeit von L-Arabinose zu L-Ribose (vgl. tschechisches Patent Nr. 149 472; Chemical Abstracts 81, 78 189 K).

Basierend auf der Erkenntnis wurde ein Verfahren entwickelt, bei dem D-Gluconsäure anstatt zu D-Arabonsäure zu D-Arabinose oxidiert wurde. Als Oxidationsmittel diente Hypochlorid. D-Arabinose wird dann in wäßriger Lösung in Gegenwart eines Molybdänkatalysators zu D-Ribose epimerisert. (vgl. EP 20 959). Dieses Verfahren erreicht ein Epimerisierungsverhältnis (Anteil von Ribose in einer Gleichgewichtsmischung) von nur ungefähr 25 %. Dennoch ist dieses Verfahren den zuvor beschriebenen überlegen, da hierbei kein Quecksilber verwendet wird und weniger Verfahrensschritte notwendig sind. In einer Verfahrensvariante wird ein Großteil der Arabinose kristallin abgetrennt und wieder in die Epimerisierung zurückgeführt.

Um die Abtrennung der Molybdänsäure aus der Epimerisierungslösung zu erleichtern, wurde die Verwendung eines Molybdänsäure-tragenden Ionentauscher-Harzes anstelle der Molybdänsäure (vgl. US 4,029,878 oder DE 2 622 316) oder die Verwendung einer Molybdänsäure tragenden Ionentauscher-Faser, (vgl. japanische offengelegte Patentanmeldung Nr. 76 894/1980), beschrieben. Das Epimerisierungsverhältnis von D-Arabinose/D-Ribose beträgt 69,4/30,6. Die japanischen offengelegten Patentanmeldungen Nr. 54 197/1982, 54 198/1982 offenbaren ein Epimerisierungsverhältnis von 27,2 % D-Ribose. In den beschriebenen Verfahren wird mit wäßrigen Zuckerlösungen gearbeitet.

Es ist weiterhin bekannt, daß durch Erhitzen von L-Arabinose in Dimethylformamid in Gegenwart von Dioxo-bis(2,4-pentadionato-0,0′)--molybdän (VI) eine Epimerisierung von 36 % der L-Arabinose zu L-Ribose erfolgt (vgl. Abe et al. in Chemical und Pharmaceutical Bulletin, 28 (1980). Seite 1324).

Eine weitere Verbesserung der Riboseselektivität wurde dadurch erreicht, daß man Borverbindungen in 2 - 3fach molarer Menge dem Epimerisierungsgemisch zusetzte (vgl. DE-A-34 37 571). In wäßriger Lösung wurde hierbei ein Epimerisierungsgleichgewicht von ca. 60 %, in nichtwäßriger Lösung von bis zu 94 % erreicht. Die Verwendung von zahlreichen nichtwäßrigen Lösungsmitteln wurde hierbei durch die Bildung von in diesen Lösungsmitteln löslichen Borsäure-Zucker-Komplexen möglich. Diese Verfahrensvariante ist jedoch für ein technisches Verfahren wenig geeignet, da die Borsäure nicht auf eine für die Vitamin-$B_2$-Herstellung tolerierbare Menge abgetrennt werden kann, ohne daß Ribose und Arabinose mit entfernt werden, d. h., daß die Ausbeute an Gesamtzuckern mit jeder Maßnahme zur Abtrennung der Borsäure stark abfällt. Außerdem kann die unumgesetzte Arabinose aus borsäurehaltiger Lösung nur unvollständig und unter Verlusten von Ribose abgetrennt und wieder eingesetzt werden. Auch die chromatographische Abtrennung der Borsäure verläuft wenig vorteilhaft, da 1) die Trennkapazität bezogen auf Ribose durch die große Borsäuremenge erheblich gemindert wird und 2) die Borsäure eine starke Verbreiterung des Ribosepeaks verursacht, wodurch das Ribose Eluat in stark verdünnter Form anfällt.

Bei den oben beschriebenen Verfahren wurde jeweils der Umsatz von Arabinose optimiert. Im Falle des an Ionenaustauscher gebundenen Katalysators entfällt zusätzlich die Abtrennung des Katalysators. Bei allen Verfahren entstehen jedoch Nebenprodukte. Das sind z.B. bei der Epimerisierung von Arabinose in kleinerer Menge die 3-Epimere Lyxose und Xylose sowie in größerer Menge nicht reduzierende Zucker-Folgeprodukte.

Die in den japanisch offengelegten Patentanmeldungen 54 197/1982 und 54 198/1982 beschriebene Produktverteilung beträgt 25,1 % Ribose, 66,4 % Arabinose, 0,6 % Lyxose und Xylose sowie 7,9 % nicht reduzierende Nebenprodukte.

Für ein optimales Verfahren zur Herstellung von D-Ribose genügt es jedoch nicht, wenn lediglich der Riboseanteil im Pentosengemisch möglichst hoch ist. Da die nicht umgesetzte Arabinose in der Regel abgetrennt und in den Epimerisierungscyclus zurückgeführt wird, ist es mindestens ebenso wichtig, daß die Bildung von nicht reduzierenden Nebenprodukten und von Lyxose und Xylose möglichst klein gehalten wird. Je mehr unerwünschte Nebenprodukte entstehen, desto schwieriger wird eine Arabinoserückführung und desto geringer wird die Riboseselektivität. Werden die Nebenprodukte nach der Abtrennung der Hauptmenge an uneingesetzter Arabinose durch Kristallisation aus der Mutterlauge zusammen mit dem Produkt Ribose ausgeschleust, sinkt bei der Vitamin-$B_2$-Herstellung in der Folgestufe die Ausbeute an N-D-Ribityl-3,4-Xylidin (vgl. Merck-Patent EP-B-20 959).

Es war daher die Aufgabe der Erfindung, das Verfahren zur Herstellung einer D-Ribose-haltigen Lösung aus einer D-Arabinose-haltigen Lösung durch Epimerisierung in Gegenwart einer Molybdän (VI)-Verbindung so zu verbessern, daß das Epimerisierungsgleichgewicht auch ohne Anwesenheit von nennenswerten Mengen Borsäure zugunsten der Ribose verschoben wird. Es war weiterhin die Aufgabe der Erfindung, ein von den Nachteilen des Standes der Technik freies Epimerisierungsverfahren zu entwickeln, das auch für die Epimerisierung anderer Pentosen und Hexosen geeignet ist.

Es wurde nun überraschenderweise gefunden, daß das Epimerisierungsgleichgewicht für die Epimerisierung von Arabinose zu Ribose auch in Abwesenheit von nennenswerten Mengen der schlecht wieder abtrennbaren Borsäure und auch ohne die Verwendung von ganz speziellen Molybdänverbindungen, wie dem Dioxi-bis-(2,4-pentandionato-0,0′-)-molybdän-(VI) in Dimethylformamid zugunsten der D-Ribose verschoben und eine Nebenproduktbildung weitgehend verhindert werden kann, wenn man die Epimerisierung in Gegenwart eines mit einer Molybdän (VI)-Verbindung beladenen basischen Ionenaustauschers in einem Gemisch aus Wasser und Methanol oder Ethanol bei Temperaturen von 70 bis 100 °C, vorzugsweise 75 bis 80 °C durchführt. Das Verfahren läßt sich technisch mit Vorteil kontinuierlich durchführen und ist auch auf die Epimerisierung anderer D- und L-Zucker mit trans-ständigen OH-Gruppen in 2- und 3-Stellung anwendbar.

Gegenstand der Erfindung ist daher ein kontinuierliches Verfahren zur Epimerisierung von Pentosen und Hexosen durch Erhitzen von in einem Lösungsmittel gelöstem Zucker in Gegenwart einer Molybdän-(VI)-Verbindung, dadurch gekennzeichnet, daß man zur Herstellung eines D- oder L-Zuckers der Formeln Ia oder Ib mit cis-ständigen OH-Gruppen in 2- und 3-Stellung des Zuckers

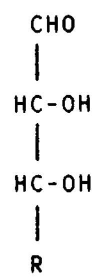

(Ia)

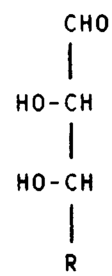

(Ib)

in der R für einen der Reste

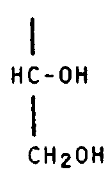

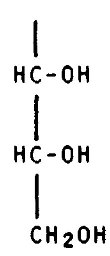

oder

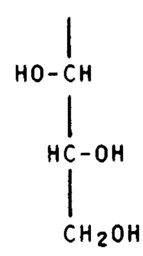

steht,

eine homogene Lösung des entsprechenden Zuckers der Formeln IIa oder IIb mit trans-ständigen OH-Gruppen in 2- und 3-Stellung des Zuckers

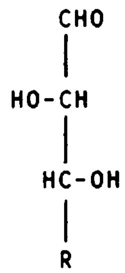

(IIa)

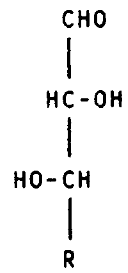

(IIb)

in einem Gemisch aus Wasser und Methanol oder Ethanol bei Temperaturen von 70 bis 100 °C , vorzugsweise 73 bis 80 °C , kontinuierlich durch ein Reaktionsrohr leitet, das den mit der Molybdän(VI)-Verbindung beladenen basischen Anionenaustauscher enthält.

Mit Hilfe dieses Verfahrens kann beispielsweise eine etwa 35 Gew.-%ige Lösung von Arabinose in Methanol/Wasser = 1/1 mit einem Umsatz von 35 % und einer Riboseselektivität von 91 % zu D-Ribose epimerisiert werden, ohne daß nachweisbare Mengen an D-Lyxose oder Xylose gebildet werden. Der Zuckerverlust beträgt nur etwa 3 % beträgt. Die so erhaltene Produktlösung ist nur schwach gelb gefärbt. Aus ihr kann durch Einengen auf ca. 70 % Trockensubstanz ein Großteil der unumgesetzten Arabinose in mindestens 99 %iger Reinheit auskristallisiert und in die Epimerisierung zurückgeführt werden.

Als Katalysator für die Epimerisierung wird ein mit Molybdän(VI)-Verbindung beladener basischer Ionenaustauscher verwendet. Der Einsatz solcher Katalysatoren ist an sich bekannt (vgl. JP-A-54 197/1982). Als Ionenaustauscher eignen sich sowohl schwach basische, wie Lewatit MP62, als auch stark basische, wie Lewatit MP 500. Mit beiden Ionenaustauschertypen wurden hervorragende Ergebnisse erzielt.

Das Beladen des Ionenaustauschers kann beispielsweise so erfolgen, wie es in Carbohydrate Research 153 (1986), Seiten 263-270, besonders Seite 270 beschrieben ist, nämlich durch Auftragen einer 0,1 m wäßrigen Lösung von $(NH_4)_6MO_7O_{24}$ auf die Säule bis keine $Cl'$-Ionen mehr im Eluat nachweisbar sind.

Das Harz wird dann mit Wasser und anschließend mit dem für die Epimerisierung verwendeten Lösungsmittel gewaschen. Man kann aber auch von der billigeren Mo-Verbindungen $MoO_3$ und $H_2MoO_4$ ausgehen, die in wäßriger Lösung mit Ammoniak oder $Na_2MoO_4$ aufgelöst werden können. Durch Einstellen eines pH-Wertes von 3-7, vorzugsweise 5-6 kann hieraus eine entsprechende Paramolybdatlösung hergestellt werden. Das Ionenaustauscherharz wird so mit etwa 1 Mol Mo/L Harz beladen.

Aus dem so hergestellten Katalysator werden bei der Epimerisierung nur geringe Spuren von Molybdat ausgewaschen, die teilweise mit dem Arabinosekristallisat letztlich wieder auf den Austauscher gelangen und teilweise bei der chromatographischen Reinigung der Mutterlauge der Arabinosekristallisation von der Ribose abgetrennt werden.

Die Epimerisierung wird in Gemischen aus Wasser und Methanol oder Ethanol durchgeführt. Wird nur mit Wasser als Lösungsmittel gearbeitet, ist der Umsatz für ein technisches Verfahren nicht hoch genug. Der Umsatz ist umso größer, je höher der Alkoholgehalt ist. Der Umsatz steigt in der Reihenfolge: Wasser < Methanol < Ethanol. Bei der Wahl des Lösungsmittels muß aber auch berücksichtigt werden, daß die Löslichkeit der Arabinose in der Reihenfolge Wasser > Methanol > Ethanol sinkt. Für eine technische Anlage ist eine möglichst hohe Produktkonzentration wichtig. Andererseits muß sichergestellt sein, daß in den Leitung keine Arabinose auskristallisiert. Mögliche Verhältnisse für Methanol/Wasser sind 95/5 bis etwa 30 : 70, vorzugsweise 80/20 bis 40 : 60, insbesondere etwa 50 : 50. Bei Verwendung von Methanol/Wasser = 50/50 kann beispielsweise mit einer 35 %igen Zuckerlösung gearbeitet werden, die auch bei unzureichender Isolation und geringer Lineargeschwindigkeit der Lösung weit genug vom Kristallisationspunkt entfernt ist. Bei Verwendung von Ethanol ist etwas mehr Wasser im Gemisch notwendig. Mögliche Verhältnisse von Ethanol/Wasser sind 85/15 bis 30/70, vorzugsweise 70/30 bis 50/50.

Die Verweilzeit der Zuckerlösung am Katalysator beträgt im allgemeinen etwa 30 bis 120 Minuten, vorzugsweise 60 bis 80 Minuten. Sie ist abhängig von der Reaktionstemperatur. Ganz allgemein kann man sagen, daß man bei kürzeren Verweilzeiten Temperaturen von über 80 °C anwendet, während bei Temperaturen unterhalb 80 °C längere Verweilzeiten nötig sind. Ein großer Vorteil des erfindungsgemäßen Verfahrens liegt darin, daß man auch bei den bevorzugten niedrigen Temperaturen von 73 bis 80 °C, bei

denen die Nebenproduktbildung extrem gering ist und die gängigen Ionenaustauscher auch im Dauerbetrieb ohne Schädigung arbeiten, nicht übermäßig lange Verweilzeiten benötigt. Zu lange Verweilzeiten führen zur Erhöhung des Zuckerverlustes und zum Auftreten von Lyxose und Xylose, d.h. vermindern die Selektivität und Lebensdauer des Kontaktes. Die Verweilzeit bei kontinuierlicher Verfahrensführung wird bei gegebener Reaktorlänge mittels der Durchflußgeschwindigkeit gesteuert.

Das erfindungsgemäße Epimerisierungsverfahren ist anwendbar für folgende Epimerisierungsgleichgewichte:

1) Arabinose (IIa) → Ribose (Ia)
2) Xylose (IIb) → Lyxose (Ib)
3) Glucose (IIb) → Mannose (Ib)
4) Galactose (IIb) → Talose (Ib)
5) Altrose (IIa) → Allose (Ia)
6) Idose (IIa) → Gulose (Ia)

wobei die Epimerisierung von Altrose und Idose weniger wichtig ist, da diese Zucker selbst nur schlecht zugänglich sind.

Ganz besondere Bedeutung aber kommt dem erfindungsgemäßen Verfahren zu, wenn man zur Herstellung einer überwiegend D-Ribose-enthaltenden Lösung eine D-Arabinose enthaltende Lösung epimerisiert, da einerseits Ribose ein besonders begehrter Zucker ist, und andererseits bei dieser Epimerisierung die größten Vorteile erzielt werden.

Gegenstand der Erfindung ist daher auch ein kontinuierliches Verfahren zur Epimerisierung von D- und L-Zuckern durch Erhitzen von in einem Lösungsmittel gelöster D-Arabinose in Gegenwart einer Molybdän-(VI)-Verbindung, das dadurch gekennzeichnet ist, daß man zur Herstellung von D-Ribose eine homogene Lösung von D-Arabinose in einem Gemisch aus Wasser und Methanol oder Ethanol bei Temperaturen von 70 bis 100 °C, vorzugsweise 73 bis 80 °C kontinuierlich durch ein Reaktionsrohr leitet, das den mit der Molybdän(VI)-Verbindung beladenen basischen Anionenaustauscher enthält.

In seiner bevorzugten Ausführungsform wird das erfindungsgemäße Verfahren auf folgende Weise durchgeführt. Eine etwa 30 bis 40 Gew.-%ige wäßrig-alkoholische Zuckerlösung wird bei 80 bis 85 °C über das mit dem basischen Ionenaustauscher in Molybdatform gefüllte Reaktionsrohr geleitet. Die aus dem Reaktionsrohr austretende Lösung wird von einem Gehalt an Trockensubstanz von ca. 40 % auf ca. 65 - 70 % kontinuierlich eingedampft. Durch Zugabe geringer Mengen an Methanol und Abkühlen auf 0 °C kristallisiert die Hauptmenge an unumgesetzter Arabinose aus, die nach Abtrennen und Waschen wieder in die Epimerisierung zurückgeführt wird.

Die nach Aufkonzentrieren etwa 65 bis 70 % Ribose enthaltende Mutterlauge wird in an sich bekannter Weise (vgl. J. Angyal et. al, Carbohydrate Research 73, (1979) 9-18 und dort zitierter Literatur) an einem stark sauren Ionenaustauscher in der $Ca^{2+}$-Form in 3 Fraktionen zerlegt, die folgende Verbindungen enthalten:

1) Nebenprodukte, einschließlich Spuren-Molybdat (Entsorgung)
2) Arabinose und Ribose, die in die Arabinosekristallisationsstufe zurückgeführt werden und
3) Ribose mit einer Reinheit von mehr als 99,9 % (?)

Das erfindungsgemäße Verfahren hat gegenüber dem bekannten Verfahren viele Vorteile.

1) Es werden keine Lyxose und Xylose gebildet, wodurch weniger Zucker verloren geht und auch keine störende Anreicherung dieser Zucker im Kreislauf bei der kontinuierlichen Verfahrensführung auftreten kann.
2) Durch die geringe Nebenproduktbildung geht weniger Arabinose verloren und braucht weniger Abfallprodukt entsorgt werden.
3) Der geringe Anteil an Nebenprodukten wird durch chromatographische Reinigung entfernt und dadurch ein Anreichern im Produktkreislauf verhindert.
4) Die gesamte Arabinose kann zurückgeführt werden, wodurch die Selektivität der Riboseherstellung steigt.
5) Die Arabinose bzw. die Ribose kristallisieren infolge ihrer hohen Reinheit nach dem Aufkonzentrieren auch aus Wasser spontan aus.
6) Durch die Verwendung von Methanol- oder Ethanol-Wassergemischen kann die Umsetzung auch schon bei Temperaturen von 73 bis 80 °C, bei denen die gängigen Anionenaustauscher auch im Dauerbetrieb ohne Beschädigung arbeiten, vorteilhaft durchgeführt werden.

Beispiele 1 und 2

Die Epimerisierung wurde in einem temperierbaren Rohr (Ø 25 mm, L = 1000 m = Schichthöhe des Ionenaustauschers) durchgeführt, durch das eine heiße Lösung von Arabinose in 1) $CH_3OH/H_2O$ (1/1) und 2) $C_2H_5OH/H_2O$ (85/15) mit Geschwindigkeiten von 5 - 10 ml/min gepumpt wurde.

Die Konzentration von reduzierbarem Zucker wurde nach Luff-Schorl ermittelt. Das Verhältnis von Ribos/Lyxose/Xylose/Arabinose wurde per HPLC bestimmt (2 hintereinandergeschaltete 30 × 0,2 mm Carbohydrate, $CH_3CN/H_2O$ = 98/12, 50 °C, 1,5 ml/min, RI).

Die präparative Chromatographie der etwa 70 % Ribose enthaltenden Mutterlauge nach Abtrennen der Hauptmenge unumgesetzter Arabinose und Einengen wurde mit einer 6,3 × 800 mm Säule bei 60 °C, 33 ml $H_2O$/min an einem stark sauren Ionenaustauscher in der $Ca^{2+}$-Form durchgeführt. Man erhielt die Ribose in einer Reinheit von mehr als 99,9 %.

Ergebnisse der kontinuierlichen Epimerisierung an Molybdat-beladenem Lewatit MP62:

| | a) $CH_3OH/H_2O$ (1/1) 80 °C; 35 Gew.%ige Lösung [%] | b) $C_2H_5OH/H_2O$ (85 : 15) 75 °C; 10 Gew.%ige Lösung [%] |
|---|---|---|
| D-Ribose | 31,9 | 37,2 |
| D-Arabinose | 65,0 | 60,0 |
| D-Lyxose/Xylose | ≤ 0,1* | ≤ 0,1* |
| Zuckerverlust | 3,1 | 2,8 |
| Umsatz | 35 | 40 |
| Selektivität | 91 | 93 |

* = nicht nachweisbar durch HPLC trotz Überladen der Säule mit Austragslösung.

## Patentansprüche

1. Kontinuierliche Verfahren zur Epimerisierung von Pentosen und Hexosen durch Erhitzen von in einem Lösungsmittel gelöstem Zucker in Gegenwart eines mit einer Molybdän(VI)-Verbindung beladenen basischen Anionenaustauschers, dadurch gekennzeichnet, daß man zur Herstellung eines D- oder L-Zuckers der Formeln Ia oder Ib mit cis-ständigen OH-Gruppen in 2- und 3-Stellung des Zuckers

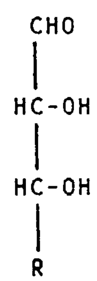

(Ia)

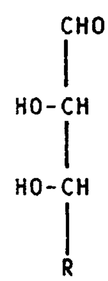

(Ib)

in der R für einen der Reste

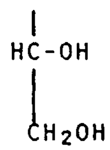

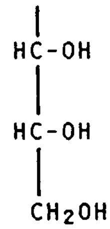

oder

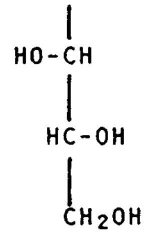

steht,
eine homogene Lösung des entsprechenden Zuckers der Formeln IIa oder IIb mit trans-ständigen OH-Gruppen in 2- und 3-Stellung des Zuckers

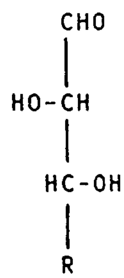

(IIa)

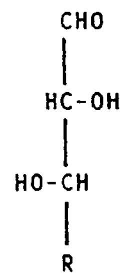

(IIb)

in einem Gemisch aus Wasser und Methanol oder Ethanol bei Temperaturen von 70 bis 100 °C kontinuierlich durch ein Reaktionsrohr leitet, das den mit der Molybdän(VI)-Verbindung beladenen basischen Anionenaustauscher enthält.

**2.** Verfahren gemäß Anspruch 1, dadurch gekennzeichnet, daß man eine Lösung des Zuckers der Formeln IIa oder IIb in einem Gemisch aus 5 bis 70 Gew.% Wasser und 95 bis 30 Gew.% Methanol erhitzt.

**3.** Verfahren gemäß Anspruch 1, dadurch gekennzeichnet, daß man die wäßrig-alkoholische Lösung des Zuckers kontinuierlich bei Temperaturen von 75 bis 80 °C durch das Reaktionsrohr leitet, das den mit der Molybdän(VI)-Verbindung beladenen basischen Anionenaustauscher enthält.

**4.** Verfahren gemäß Anspruch 1, dadurch gekennzeichnet, daß man zur Herstellung von D-Ribose eine wäßrig-alkoholische Lösung von D-Arabinose bei Temperaturen von 70 bis 100 °C durch das Reaktionsrohr leitet, das den mit der Molybdän(VI)-Verbindung beladenen basischen Anionenaustauscher enthält.

**5.** Verfahren gemäß Anspruch 1, dadurch gekennzeichnet, daß man zur Herstellung von D-Ribose eine wäßrig-alkoholische Lösung von D-Arabinose bei Temperaturen von 73 bis 80 °C durch das Reaktionsrohr leitet, das den mit der Molybdän(VI)-Verbindung beladenen basischen Anionenaustauscher enthält.

**6.** Verfahren gemäß Anspruch 4, dadurch gekennzeichnet, daß man zur Herstellung von D-Ribose
a) eine wäßrig-alkoholische Lösung von D-Arabinose bei Temperaturen von 70 bis 83 °C durch ein Reaktionsrohr leitet, das den mit der Molybdän(VI)-Verbindung beladenen basischen Ionenaustauscher enthält,
b) das erhaltene Eluat bis auf einen Gehalt von etwa 65-70 % Trockensubstanz einengt,
c) durch Zugabe von Methanol bzw. Ethanol und Abkühlen auf etwa 0 °C unumgesetzte Arabinose auskristallisieren läßt, diese abtrennt und zurückführt
d) das anfallende Filtrat nach Aufkonzentrieren in an sich bekannter Weise an einem stark sauren Ionenaustauscher in der $Ca^{2+}$-Form reinigt und

e) die hierbei erhaltene nebenproduktfreie Arabinose- D-Ribose--Misch-Fraktion in die Arabinosekristallisationsstufe zurückführt.

## Claims

1. A continuous process for epimerizing a pentose or hexose by heating a sugar in solution in a solvent in the presence of a basic anion exchanger loaded with a molybdenum(VI) compound, which comprises preparing a D- or L-sugar of the formula Ia or Ib having cis-disposed OH groups in the 2- and 3-positions of the sugar

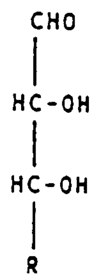

(Ia)

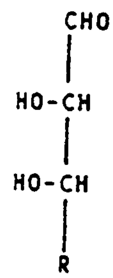

(Ib)

where R is one of the radicals

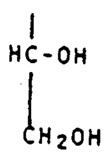

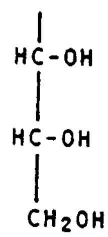

or

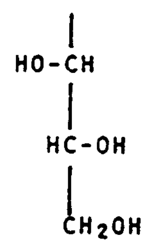

by continuously passing a homogeneous solution of the corresponding sugar of the formula IIa or IIb having transdisposed OH groups in the 2- and 3-positions of the sugar

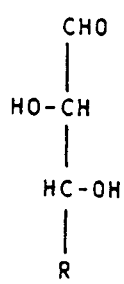

(IIa)

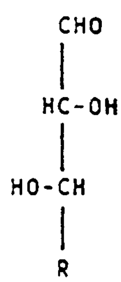

(IIb)

in a mixture of water and methanol or ethanol at from 70 to 100 ° C through a reaction tube which contains the basic anion exchanger loaded with the molybdenum(VI) compound.

2. A process as claimed in claim 1, wherein a solution of the sugar of the formula IIa or IIb is heated in a mixture of from 5 to 70% by weight of water and from 95 to 30% by weight of methanol.

3. A process as claimed in claim 1, wherein the aqueous/alcoholic solution of the sugar is continuously passed at from 75 to 80 ° C through the reaction tube which contains the basic anion exchanger loaded with the molybdenum(VI) compound.

4. A process as claimed in claim 1, wherein, to prepare D-ribose, an aqueous/alcoholic solution of D-arabinose is passed at from 70 to 100 °C through the reaction tube which contains the basic anion exchanger loaded with the molybdenum(VI) compound.

5. A process as claimed in claim 1, wherein, to prepare D-ribose, an aqueous/alcoholic solution of D-arabinose is passed at from 73 to 80 °C through the reaction tube which contains the basic anion exchanger loaded with the molybdenum(VI) compound.

6. A process as claimed in claim 4, wherein to prepare D-ribose
   a) an aqueous/alcoholic solution of D-arabinose is passed at from 70 to 83 °C through a reaction tube which contains the basic ion exchanger loaded with the molybdenum(VI) compound,
   b) the eluate obtained is concentrated to a dry substance content of about 65-70%,
   c) methanol or ethanol is added and the mixture is cooled down to about 0 °C to crystallize out unconverted arabinose which is separated off and recycled,
   d) the filtrate obtained is concentrated and purified in a conventional manner over a strongly acid ion exchanger in the $Ca^{2+}$ form and
   e) the byproduct-free arabinose/D-ribose mixed fraction obtained is recycled into the arabinose crystallization stage.

## Revendications

1. Procédé continu d'épimérisation de pentoses et d'hexoses par chauffage de sucres dissous dans un solvant en présence d'un échangeur d'anions basique chargé d'un composé de molybdène(VI), caractérisé en ce que, pour la préparation d'un D- ou L-sucre de formule Ia ou Ib contenant des groupements OH en configuration cis dans les positions 2 et 3 du sucre

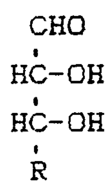
(Ia)

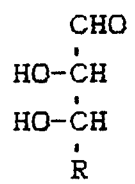
(Ib)

où R est mis pour l'un des restes

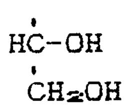

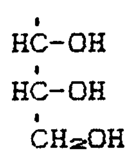

ou

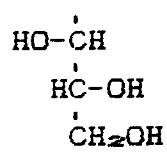

on envoie en continu une solution homogène, dans un mélange d'eau et de méthanol ou d'éthanol, du sucre correspondant de formule IIa ou IIb, contenant des groupements OH en configuration trans en positions 2 et 3 du sucre

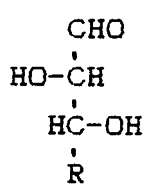
(IIa)

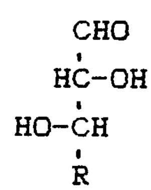
(IIb)

à des températures de 70 à 100°C, à travers un tube de réaction qui contient l'échangeur d'anions basique chargé du composé de molybdène(VI).

**2.** Procédé selon la revendication 1, caractérisé en ce qu'on chauffe une solution du sucre de formule IIa ou IIb dans un mélange de 5 à 70% en poids d'eau et de 95 à 30% en poids de méthanol.

**3.** Procédé selon la revendication 1, caractérisé en ce qu'on envoie en continu la solution hydroalcoolique du sucre, à des températures de 75 à 80°C, à travers le tube de réaction qui contient l'échangeur d'anions basique chargé du composé de molybdène(VI).

**4.** Procédé selon la revendication 1, caractérisé en ce que, pour la préparation de D-ribose, on envoie une solution hydroalcoolique de D-arabinose, à des températures de 70 à 100°C, à travers le tube de réaction qui contient l'échangeur d'anions basique chargé du composé de molybdène(VI).

**5.** Procédé selon la revendication 1, caractérisé en ce que, pour la préparation de D-ribose, on envoie une solution hydroalcoolique de D-arabinose, à des températures de 73 a 80°C, à travers le tube de réaction qui contient l'échangeur d'anions basique chargé du composé de molybdène(VI).

**6.** Procédé selon la revendication 4, caractérisé en ce que, pour la préparation de D-ribose,

a) on envoie en continu une solution hydroalcoolique de D-arabinose, à des températures de 70 à 83°C, à travers un tube de réaction qui contient l'échangeur d'anions basique chargé du composé de molybdène(VI),

b) on concentre l'éluat obtenu à une teneur d'environ 65-70% de substance sèche,

c) par addition de méthanol ou d'éthanol et refroidissement à 0°C environ, on provoque la séparation à l'état cristallin de l'arabinose n'ayant pas réagi, on sépare ce dernier et on le recycle,

d) après concentration, on purifie le filtrat obtenu, de façon connue en soi, sur un échangeur d'ions fortement acide sous la forme $Ca^{2+}$, et

e) on renvoie dans l'étage de cristallisation de l'arabinose la fraction mixte arabinose/D-ribose exempte de produits secondaires qui est ainsi obtenue.